# EUROPEAN PATENT APPLICATION

(11) **EP 4 166 084 A1**
(43) Date of publication of application: **19.04.2023**
(21) Application number: 21202960.7
(22) Date of filing: 15.10.2021
(51) Int. Cl.: A61B 5/372, A61B 5/00, A61M 21/00

(54) **SLEEP-BASED DETECTION AND INTERVENTION SYSTEM**

(71) Applicant: Universität Bern, 3012 Bern (CH)
(72) Inventor: Nissen, Christoph, 3006 Bern (CH); Fehér, Kristoffer, 3072 Ostermundigen (CH); Ruch, Simon, 72070 Tübingen (DE); Senn, Walter Martin, 3014 Bern (CH)
(74) Representative: Latscha Schöllhorn Partner AG

(57) **Abstract**

A sleep-based detection and intervention system (1) for the treatment of major depressive disorder comprises an electroencephalography cap (5) with multiple electrodes (6a, 6b, 6c, 6d, 6e, 6f, 6g, 6h) configured for recording an electroencephalogram of a patient's brain, an earphone device (4) configured for one ear or the ears of the patient (7) and a processing unit (2) being connected to the electroencephalography cap (5) and to the earphone device (4). The processing unit (2) includes a template-based algorithm (33) configured for detecting slow wave sleep of the patient (7) based on the electroencephalogram recorded by the multiple electrodes (6a, 6b, 6c, 6d, 6e, 6f, 6g, 6h) of the electroencephalography cap (5), and an acoustic intervention protocol (8) configured for providing a noise stimulation to the patient (7) via the earphone device (4) when slow wave sleep is detected by the template-based algorithm (33).

## Description

### Technical Field

The present invention relates to a sleep-based detection and intervention system for the treatment of major depressive disorder (MDD).

### Background Art

MDD is listed by the World Health Organization as the leading cause for illness-related reduction of quality of life worldwide. Therapeutic sleep deprivation has been known since the 1970s to exert a strong and rapid antidepressant effect on individuals with MDD. However, the effect is short-lived, with most patients relapsing following a period of subsequent sleep. Furthermore, the burden on the patient of staying awake for an entire night is a significant limitation of the treatment.

The disclosure of WO 2018/104459 A1 pertains to determining the timing of stimulation provided to a subject during sleep. The system receives a raw signal carrying information related to slow wave activity; buffers a portion of the raw signal; determines a timing of slow wave events in the buffered portion of the raw signal; filters the raw signal; determines a timing of slow wave events in the filtered raw signal; compares the timing of the slow wave events in the buffered portion of the raw signal to the timing of the slow wave events in the filtered raw signal; determines a first correction factor associated with reducing slow wave activity in the subject and a second correction factor associated with enhancing slow wave activity in the subject; and adjusts a timing of the stimulation provided to the subject during the sleep session based on the first and/or second correction factors. It is further disclosed that delivering auditory and/or other stimulation during slow wave sleep influences the amplitude of subsequent sleep slow waves. The nature of the influence depends on the timing of the stimulation with respect to the sleep slow wave phase and/or characteristics. When the stimulation happens in the vicinity of a slow wave positive peak the subsequent slow waves have a higher amplitude (e.g., they are enhanced). The enhancement effect is believed to be beneficial for increasing the restorative value of sleep. Delivering the stimulation in the vicinity of a slow wave negative peak may have a reducing effect (e.g., a decrease in amplitude) on the amplitude of the subsequent positive peak. This effect may be useful in situations where deprivation of slow wave sleep is sought, as for example in the framework of depression relief.

However, there are still shortcomings regarding the accuracy with which slow wave sleep may be detected and regarding effective interventions at the patient as a consequence of the slow wave sleep detection, in particular with respect to the treatment of MDD.

It is therefore the object of the present invention to provide for a high accuracy sleep-based detection and intervention system and a respective method of sleep-based detection and intervention which is less stressful for the patient.

### Disclosure of the Invention

According to the invention these needs are settled by a sleep-based detection and intervention system for treatment of MDD. The system comprises an electroencephalography (EEG) cap with multiple electrodes configured for recording an EEG of a patient's brain, an earphone device configured for one ear or the ears of the patient (i.e. the earphone device may be placed in or onto one or both ears of the patient) and a processing unit being connected to the EEG cap and to the earphone device. The processing unit includes a template-based algorithm configured for detecting slow wave sleep of the patient based on the EEG recorded by the multiple electrodes of the EEG cap, and an acoustic intervention protocol configured for providing a noise stimulation to the patient via the earphone device when slow wave sleep is detected by the template-based algorithm.

Generally, EEG is an electrophysiological monitoring method to record electrical activity on the scalp that has been shown to represent the macroscopic activity of the surface layer of the brain underneath (brain activity). It is typically non-invasive, with the electrodes placed along the scalp. It is noted that EEG may also be used to diagnose epilepsy, which causes abnormalities in EEG readings, but also to diagnose sleep disorders, depth of anesthesia, coma, encephalopathies, and brain death.

The term "electrodes" as used herein includes all types of measuring units and/or sensors which are capable of measuring brain activities respectively the corresponding voltage values.

It is noted that the electrode placement sites on the head respectively scalp generally refer to the lobe or area of the brain they are reading from. The electronic placement sites are commonly designated frontal (F), temporal (T), parietal (P), occipital (O) and central (C).

The term "processing unit" as used herein may include a personal computer or a mobile communication device such as a laptop computer a tablet or a mobile phone or the like.

The term "earphone device" as used herein may include earphones, headsets, headphones, earbud headphones and the like.

The term "template-based algorithm" as used herein generally includes the means used by the processing unit primarily for the purpose of detecting slow wave sleep (SWS) of a patient.

It is noted in this respect that according to the criteria by the American academy of sleep medicine (AASM), SWS is defined as a 30-s epoch containing at least approximately 6 slow waves (SWs) of >75 mV amplitude at frontal channels.

Preferably, the template-based algorithm is based on a template, referring to averaged slow wave sleep information of multiple test persons.

The term "template" as used herein refers to this averaged slow wave sleep information. More precisely, the (average) slow wave sleep information used for creating the template is in the form of multiple whole head topographies from each of the multiple test persons recorded at the time-point of a frontal slow wave peak in the electroencephalogram.

The frontal slow wave peaks are measured by one or more electrodes of the EEG cap in the frontal region of the brain, in particular in the region of the frontal lobe. If a frontal slow wave peak occurs, the voltage values of all electrodes of the EEG cap are recorded to form the whole head topography. The whole head topography may therefore be regarded as a sort of "screen shot" of the patient's brain on the basis of the recorded voltage values.

Preferably, the template based algorithm is configured to determine a correlation of whole head topographies of the patient with the (average) template, i.e. in order to detect slow wave sleep. This approach has proven to provide a significantly improved accuracy for determining the occurrence of SWS.

Preferably, the template-based algorithm is configured to use the determined correlation of the whole head topographies of the patient with the template (i.e. as predictors) in a linear model including a first regressor and a second regressor. The linear model is preferably a linear regression model.

Preferably, the first, template-based, regressor comprises the slow oscillation (SO) power of the template correlation, the variance of the slow oscillation (SO) power of the template correlation and a relative percentage of the slow oscillation (SO) power of the template correlation. EEG activity in the SO frequency is generally considered as hallmarks of the EEG during slow wave sleep. SO refers to a rhythmic activity (0.5-2 Hz). The individual waves constituting this activity are called slow waves (SWs).

Preferably, the second regressor includes the average global gamma power of the electroencephalogram. The average global gamma power represents the average gamma power measured by the electrodes of the EEG cap across the whole head (all channels) in a frequency range of about 30 Hz to about 40 Hz.

Preferably, the correlation of the whole head topographies of the patient with the template is calculated over a moving window, respectively, wherein the slow oscillation power of the template correlation, the variance of SO power of the template correlation and the percentage of power in SO frequency band are preferably calculated across a 10 s moving window and wherein the average global gamma power of the encephalogram is preferably calculated across a 4 s moving window. Herewith, an efficient calculation of the correlation is ensured.

Preferably, the template-based algorithm is configured to determine the presence of artifacts/arousals based on an (additional) artifact/arousal detection process which runs parallel to the SWS detection. Artifacts/arousals may for instance include blink of an eye, eye movements, swallowing, muscle twitching, pulse and poorly fitting electrodes. In this manner, any corruption of the measuring results may be avoided. In other words, when an artifact/arousal is detected by the artifact/arousal process, this negates a (positive) decision of the SWS detection process (i.e. which is carried out by the general linear model with the first regressor and the second regressor).

Preferably, the artifact/arousal detection process includes the voltage range of the electroencephalogram, the amplitude and the frontal delta power (frequency of delta brain waves measured by the frontal electrodes respectively calculated on the frontal channels of the electroencephalogram) as well as the average global gamma power (frequency of gamma brain waves measured by all electrodes respectively calculated on all channels of the electroencephalogram) of the electroencephalogram.

The frontal delta power is preferably measured in a frequency range of about 0.1 Hz and about 4 Hz. This has proven to be the most effective approach for measuring delta brain waves.

Preferably, the frontal amplitude and the frontal delta power and the average global gamma power of the electroencephalogram are determined across a 4 s moving window and checked every 0.5 s. Hereby, the accuracy of the determination scheme may be improved.

It is generally noted that delta brain waves (also called delta rhythm) represent the slowest frequency and highest amplitude brain waves, i.e. with a frequency between approximately 0.1 Hz and approximately 4 Hz, and an amplitude of approximately 100 µV to approximately 200 µV. Delta brain waves are usually associated with the deep sleep stage N3 of NREM (non-rapid eye movement) sleep, also known as slow wave sleep.

In this context, it is noted that NREM sleep generally includes three stages N1, N2 and N3. Stage N1 of NREM sleep marks the transition from wakefulness to sleep. This stage typically lasts less than 10 minutes and is marked by a slowing of your heartbeat, breathing, and eye movements, as well as the relaxation of your muscles. Stage N2 of NREM sleep is a period of light sleep before you enter deeper sleep and lasts roughly 20 minutes. Stage N2 is characterized by further slowing of both the heartbeat and breathing, and the brain begins to produce bursts of rapid, rhythmic brain wave activity known as sleep spindles. As mentioned before, stage N3 of NREM sleep is the deepest period of sleep and lasts for about 20 to 40 minutes. The heartbeat and breathing slow down to their lowest levels, and the muscles are relaxed such that it may be hard to awaken a person in this stage of sleep.

Further, it is generally noted that gamma brain waves (also called gamma rhythm) have the highest frequency among all brain waves, generally between 25 Hz and 140 Hz (wherein the region between about 30 Hz and about 40 Hz being presently of particular interest). Gamma brain waves are generally associated with large scale brain network activity and cognitive phenomena such as working memory, attention, and perceptual grouping.

Preferably, the acoustic intervention protocol provides for randomized noise stimulation. In particular, pink noise is used for the noise stimulation. Pink noise is a signal or process with a frequency spectrum such that the power spectral density (power per frequency interval) is inversely proportional to the frequency of the signal. In pink noise, each octave interval (halving or doubling in frequency) carries an equal amount of noise energy.

The use of white noise is however also possible herein and also the use other types of tones. It is noted in this context that white noise generally comprises a heterogeneous mixture of sound waves extending over a wide frequency range.

Preferably, the randomized noise stimulation has a randomized duration of about 50 ms to about 500 ms. This duration has proven to be particularly effective.

Preferably, the randomized noise stimulation has a randomized linear increase of volume from about 40 dB to about 106 dB in 60s until slow wave sleep is no longer detected by the template-based algorithm. This form of increase is less stressful for the patient.

Preferably, the linear increase of volume is combined with random walks between about +/- 2.5 dB. Advantageously, the random walks are configured according to an Ornstein-Uhlenbeck process. This process can be considered to be a modification of the random walk in continuous time, or Wiener process, in which the properties of the process have been changed so that there is a tendency of the walk to move back towards a central location, with a greater attraction when the process is further away from the center.

Preferably, the randomized noise stimulation has randomized interstimulus intervals of about 1 s to about 4 s (i.e. intervals without any tone).

Preferably, the acoustic intervention protocol is configured to provide that upon artifact/arousal detection noise stimulation is reset and suppressed for a period of time, preferably for about 35 s, which has proven to be a particularly effective intervention scheme.

A further aspect of the present invention comprises a method of sleep-based detection and intervention for the treatment of major depression disorder. The method comprises the following steps: placing an electroencephalography cap on the head (respectively the scalp) of a patient; placing an earphone device in or onto one ear or both ears of the patient; providing a processing unit being connected to the electroencephalography cap and to the earphone device; detecting slow wave sleep based on the electroencephalogram by a template-based algorithm comprised in the processing unit; and providing a noise stimulation via the earphone device to the patient when slow wave sleep is detected.

Preferably, upon an artifact/arousal detection the noise stimulation is reset and suppressed for a period of time, preferably for about 35 s, which has proven to be a particularly effective intervention scheme.

The improved slow wave sleep (SWS) detection approach in humans, as described herein, ensures a robust all night SWS detection across different age groups. The detection approach relies on spatial filtering that is based on the topographical distribution of potentials across the cortex during frontal slow waves. During SWS, the degree of correlation with this template will fluctuate in a rhythm typical of SWS. These rhythmic, coherent changes in correlation across channels can thereby be used as a biologically relevant and highly robust marker for SWS. Its characteristics makes it less sensitive to the changes in slow wave amplitudes within individuals (e.g. throughout the night) as well as to differences between individuals (e.g. young versus elderly, and different patient populations).

The aforementioned achievements are further supported by the new intervention protocol for disrupting SWS, involving the application of noise of increasing volume upon detection of SWS. In this respect, it is noted that the human brain is particularly sensitive to auditory input during sleep, potentially to detect distant threats in the dark. Stronger stimulation activates arousal-pathways disrupting sleep, which is capitalized on for SWS stage disruption. Of particular note, a full randomization of noise application, including duration, interstimulus interval and volume increase (random walk) is applied with the present invention for the first time.

Finally it shall be noted that it is also conceivable that the system and method according to the present invention may also be applied for the treatment of other diseases, as for example the treatment of epileptic seizures and the like.

### Brief Description of the Drawings

The sleep-based detection and intervention system according to the present invention is described in more detail herein below by way of exemplary embodiments and with reference to the attached drawings, in which:
- Fig. 1: illustrates a side view of an inventive sleep-based detection and intervention system;
- Fig. 2: illustrates the creation of a template according to the present invention;
- Fig. 3: illustrates the application of the template in accordance with the present invention;
- Fig. 4: illustrates slow wave sleep detection and suppression in accordance with the present invention;
- Fig. 5: depicts an acoustic noise stimulation in accordance with the present invention; and
- Fig. 6: depicts an example of slow wave sleep suppression in accordance with the present invention.

### Description of Embodiments

In the following description certain terms are used for reasons of convenience and are not intended to limit the invention. The terms "right", "left", "up", "down", "under" and "above" refer to directions in the figures. The terminology comprises the explicitly mentioned terms as well as their derivations and terms with a similar meaning. Also, spatially relative terms, such as "beneath", "below", "lower", "above", "upper", "proximal", "distal", and the like, may be used to describe one element's or feature's relationship to another element or feature as illustrated in the figures. These spatially relative terms are intended to encompass different positions and orientations of the devices in use or operation in addition to the position and orientation shown in the figures. For example, if a device in the figures is turned over, elements described as "below" or "beneath" other elements or features would then be "above" or "over" the other elements or features. Thus, the exemplary term "below" can encompass both positions and orientations of above and below. The devices may be otherwise oriented (rotated 90 degrees or at other orientations), and the spatially relative descriptors used herein interpreted accordingly. Likewise, descriptions of movement along and around various axes include various special device positions and orientations.

To avoid repetition in the figures and the descriptions of the various aspects and illustrative embodiments, it should be understood that many features are common to many aspects and embodiments. Omission of an aspect from a description or figure does not imply that the aspect is missing from embodiments that incorporate that aspect. Instead, the aspect may have been omitted for clarity and to avoid prolix description. In this context, the following applies to the rest of this description: If, in order to clarify the drawings, a figure contains reference signs which are not explained in the directly associated part of the description, then it is referred to previous or following description sections. Further, for reason of lucidity, if in a drawing not all features of a part are provided with reference signs it is referred to other drawings showing the same part. Like numbers in two or more figures represent the same or similar elements.

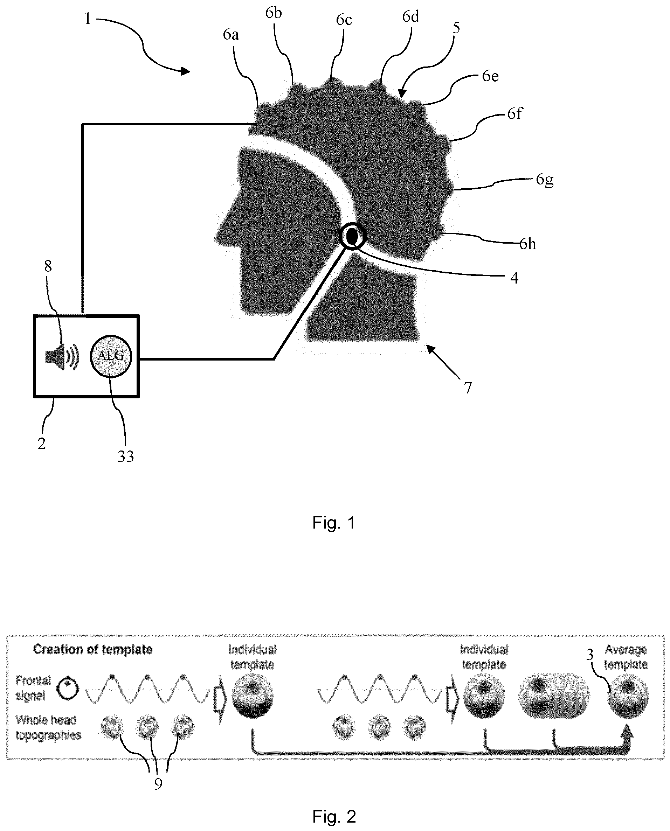

**Fig. 1** shows a schematic illustration of an inventive sleep-based detection and intervention system 1 for treatment of major depressive disorder. The system comprises an EEG cap 5 with multiple electrodes 6a, 6b, 6c, 6d, 6e, 6f, 6g, 6h configured for recording an electroencephalogram of a patient's brain wherein the electrodes detect electric potential differences at the scalp of the patient 7. Thereby, electrodes 6a and 6b are for example placed in the frontal region, electrodes 6c and 6d in the central region, electrodes 6e and 6f in the parietal region and electrodes 6g and 6h in the occipital region. The electrodes at the lateral head portions (i.e. left temporal and right temporal regions) are not illustrated herein.

Earphone device 4 is configured to be placed in or onto one or both ears of the patient 7 and to provide for acoustic noise when activated. The earphone device 4 may be in the form of earbuds, earphones or a headset or the like.

The system 1 further comprises a processing unit 2 which is connected to the EEG cap 5 and to the earphone device 4. The processing unit 2 includes a template-based algorithm 33 configured for detecting slow wave sleep of the patient 7 based on the EEG recorded by the multiple electrodes 6a, 6b, 6c, 6d, 6e, 6f, 6g, 6h of the EEG cap 5, and an acoustic intervention protocol 8 configured for providing a noise stimulation to the patient 7 via the earphone device 4 when slow wave sleep is detected by the template-based algorithm 33.

In **Fig. 2****,** the creation of a template 3 in accordance with the present invention is schematically illustrated. Frontal signals in the form of frontal slow waves, in particular fronto-central slow waves, of multiple test persons are detected. Thereby, whole head topographies 9 at the time-point of a frontal slow wave peak (cf. red points in the graph, respectively) are recorded, averaged across all instances, and normalized to create a (spatial) template of frontal slow wave peaks per individual (individual template). Finally, an average template is created across all individual test persons which is herein referred to as the template 3.

It is noted that for the template creation only N3 sleep was used, and only the first or the first two NREM cycles of the night were used for creating the templates (selected manually, but with a mean time lag of ∼2.5 h from lights off). Namely, slow waves can change drastically with respect to wave-form, amplitude and topographic distribution across the night. Focusing on only the first sleep cycles would reduce the inter-subject variability of the maps.

Since slow waves show a typical fronto-central maximum in the power spectrum, the detection of slow waves was limited to the fronto-central channels that during slow wave sleep express the largest power in the slow wave frequency range of 0.5 Hz - 2 Hz (the power in the slow wave frequency range is also referred to as SO power or slow oscillation power). An average signal was calculated for six frontal electrodes. The EEG was bandpass filtered between 0.15 Hz and 2 Hz. For the identification of slow waves, time points of positive to negative zero crossings were identified in the signal. The lowest and highest value between every 2 of these time points were detected (i.e. one negative and one positive peak between two succeeding positive to negative zero crossings). Intervals of positive to negative zero crossings with a length of 0.9 s - 2 s were marked as slow wave epochs if the corresponding amplitude difference between peaks (positive peak minus negative peak) was > 2/3 of the standard deviation of amplitudes from the mean of amplitudes.

Averages of EEG potentials at the peak of the positive and the negative half-wave of all detected SWs were calculated per EEG channel. A prototypical voltage distribution (also described as a topographic or spatial template) of frontal slow wave peaks and troughs respectively was created per individual. Grand mean averages across all subjects were calculated per EEG channel to create a template across all individuals. Finally, the template was normalized by its maximum absolute value, i.e. by dividing each element of the vector by the vector's maximum absolute value.

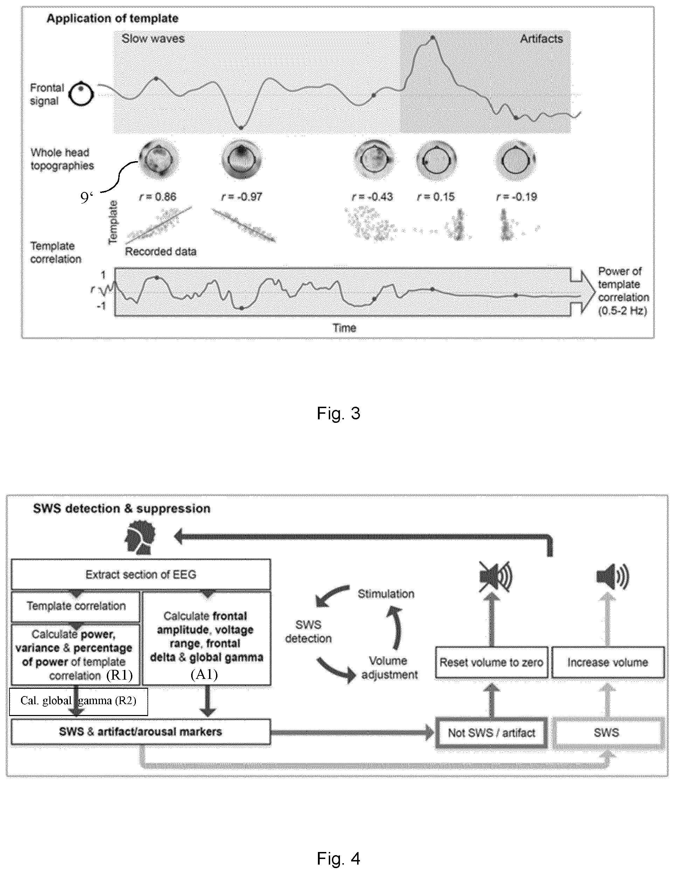

In **Fig. 3****,** the application of the template according to the present invention is schematically illustrated. To detect slow wave sleep, whole head topographies 9' of the patient 7 are correlated with the template 3. Whole head potentials 9' correlate positively with the template upon frontal slow wave peaks, negatively upon troughs, while correlation decreases between peaks and troughs or for artifacts. The power of the, for slow waves distinctive ∼ 1 Hz rhythmic fluctuation of the correlation over time, marks the presence of slow wave sleep. As it is a correlation, it is insensitive to the amplitude of recorded potentials. This is advantageous firstly as different individuals have different amplitudes of slow waves. In particular, the amplitude of slow waves decline with age. Second, it makes the detection insensitive to huge artifacts, in particular if detection is turned on during wakefulness when the subject respectively the patient is still moving around (cf. section "Artifacts" in the graph).

The template correlation shows to what extent the brain state of the patient (measured as whole head topography 9') approaches, in a certain moment, the template (which reflects the frontal up-state). In case of the left red dot (frontal up-state) there is template correlation (p = 0.86 (positive correlation)) but not with the other red dots. In the down-state (second left red dot) the correlation is just opposite (i.e. p = - 0.97 (negative correlation)). Regarding the medium red dot (p = -0.43) there is less template correlation (since this is a different phase of the wave) and with regard to the artifacts (p = 0.15 and p = -0.19) there is likewise little template correlation.

The point-by-point correlation over time is shown in the graph at the bottom of **Fig. 3****.** Here, a further spectral analysis occurs which results in the power of template correlation (0.5 - 2 Hz). It is important to note that only if over a longer period of time a high power is measured and other conditions are met (cf. below in connection with Fig. 4) the acoustic noise stimulation is triggered. The idea here is that the noise stimulation shall not start when (only) a single slow wave occurs (this is also possible in case of lighter sleep) but only if real deep sleep is present. In doing so, slow wave sleep detection becomes very robust.

In other words, the sleep state of slow wave sleep is defined herein according to the presence of a certain amount of slow waves within an extended period. It was found that a 10 s moving window struck a good balance between reliably detecting a change into slow wave sleep, while also being reactive to changes (it is noted that a longer time-window would react slower to changes in the sleep state).

**Fig. 4** schematically depicts slow wave sleep detection and closed-loop suppression through acoustic stimulation. The template correlation is calculated over a moving window of EEG signal. The power, variance of power and percentage of power are used together with global gamma power to detect slow wave sleep. Frontal amplitude, voltage range and delta power together with global gamma power are used as artifact/arousal markers for quick suppression of stimulation.

In particular, linear regression model with data from the 10 s moving window (moving in 0.5 s steps) - i.e. an extracted section of the EEG data - was used to detect slow wave sleep. Two regressors R1 and R2 are used herein for detecting slow wave sleep which are based on the correlation with the template (first, template-based, regressor R1) as well as on gamma power of the EEG data (second, EEG-based, regressor R2).

For the first (template-based) regressor R1, the following three characteristics of the template correlation across the 10 s moving window were selected to be used as a regressor to identify a period as slow wave sleep. The first (template-based) regressor was defined as R1 = a * b / c, wherein
- a: represents the SO power of the template correlation across 10 s (oscillation of the correlation with the template at a slow oscillation frequency (0.5 Hz - 2 Hz) would indicate slow wave sleep)
- b: represents the variance of SO power of the template correlation across 10 s (a stable oscillation of the correlation at SO frequency across the 10 s would indicate stable slow wave sleep)
- c: represents the percentage of power in the SO frequency band across 10 s (a high percentage would likewise indicate stable slow wave sleep; a periodogram power spectral density estimate of the template correlation across 10 s is used)

The second regressor R2 includes the average gamma power across all channels (across a 4 s moving window).

Visually staged adaptation as well as baseline night data from 12 healthy young participants was used to train the slow wave sleep classification and determine the regressor coefficients. The data was treated exactly as the online streamed data used for slow wave sleep detection and stimulation and was therefore minimally preprocessed. The data was re-referenced to the average across all channels. A low-pass filter at 40 Hz was applied. For calculating the frontal amplitude (see further below) the data was re-referenced to the contralateral mastoids.

When comparing the classification performance based on the training data, it was concluded that using all adaptation and all baseline nights was superior to using the individual adaptation night only, the individual adaptation night and all baseline nights, or all baseline nights only. This means that it is not necessary to use the individual adaptation night to determine the coefficients, as this would require someone always to be available to score the individual adaptation night before the first experimental night. Furthermore, transition epochs (i.e. epochs of different sleep-stages bordering each other) were not used for training. For training, data from the first N1 or N2, for a period of 2 hours was used. Indeed, using all night or late night data for training did not improve performance, as evaluated based on data from early, all night or late night data.

Lasso regression with a 5-fold cross-validation on the training data was used to obtain penalized, maximum-likelihood fitted regressor coefficients. Regressor coefficients were chosen corresponding to the lambda values with minimum expected deviance. A geometric sequence of lambda values was used.

Further, every slow wave sleep detection needs an additional fast arousal/artifacts detection process (i.e. which runs parallel to SWS detection). Sleep state detection is slow, as it is assessing a longer period; presently a 10 s moving window. In contrast, the artifact detection can evaluate more immediate changes. As can be seen in **Fig. 4** the arousal/artifact detection (A1) includes the voltage range (maximum threshold), amplitude (minimum and maximum) and delta power (minimum threshold), calculated on frontal channels as well as the average global gamma power (maximum threshold). Thereby, frontal amplitude and delta power and the average gamma power were based on a 4 s data, and checked per 0.5 s. The voltage range was based on 200 ms data, checked continuously. Upon artifact/arousal detection, stimulation was reset and suppressed for 35 s.

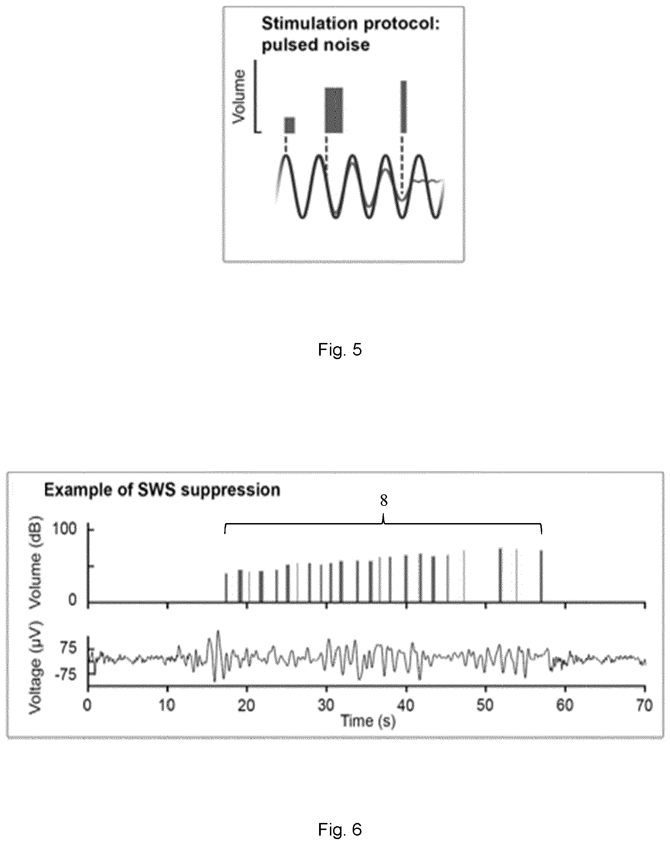

**Fig. 5** is schematical illustration of a pulsed noise stimulation protocol. Upon detection of slow wave sleep, bursts of pink noise are applied with a randomized duration and inter-onset interval. A random walk (Ornstein-Uhlenbeck process) is superimposed on a linear increase of volume, to add unpredictability in volume. In this manner slow waves may be suppressed in a particularly effective manner.

**Fig. 6** depicts one example of successful slow wave sleep suppression through pulsed noise of increasing volume in accordance with an inventive acoustic intervention protocol 8. The noise stimulation involves the application of bursts of pink noise with a randomized duration of 50 ms to 500 ms. The volume is increasing as a function of time from about 40 dB (40 dB is the lowest possible value) to about 70 dB (as highest value in this example) in about 40 s in this example, until slow wave sleep is no longer detected by the template-based algorithm. The linear increase of volume is combined with random walks between +-2.5 dB (Ornstein-Uhlenbeck process) to add unpredictability in volume. The inter-stimulus-intervals are randomized between about 1 s and about 4 s.

In this manner, the SO rhythmic activity of > 75 µV amplitude which indicates slow wave sleep (which started after about 15 s and ended after about 58 s) has successfully been suppressed.

It is again noted that according to the criteria by the American academy of sleep medicine (AASM), SWS is defined as a 30-s epoch containing at least approximately 6 slow waves (SWs) of >75 mV amplitude at frontal channels.

This description and the accompanying drawings that illustrate aspects and embodiments of the present invention should not be taken as limiting-the claims defining the protected invention. In other words, while the invention has been illustrated and described in detail in the drawings and foregoing description, such illustration and description are to be considered illustrative or exemplary and not restrictive. Various mechanical, compositional, structural, electrical, and operational changes may be made without departing from the spirit and scope of this description and the claims. In some instances, well-known circuits, structures and techniques have not been shown in detail in order not to obscure the invention. Thus, it will be understood that changes and modifications may be made by those of ordinary skill within the scope and spirit of the following claims. In particular, the present invention covers further embodiments with any combination of features from different embodiments described above and below.

The disclosure also covers all further features shown in the figures individually although they may not have been described in the afore or following description. Also, single alternatives of the embodiments described in the figures and the description and single alternatives of features thereof can be disclaimed from the subject matter of the invention or from disclosed subject matter. The disclosure comprises subject matter consisting of the features defined in the claims or the exemplary embodiments as well as subject matter comprising said features.

Furthermore, in the claims the word "comprising" does not exclude other elements or steps, and the indefinite article "a" or "an" does not exclude a plurality. A single unit or step may fulfil the functions of several features recited in the claims. The mere fact that certain measures are recited in mutually different dependent claims does not indicate that a combination of these measures cannot be used to advantage. The terms "essentially", "about", "approximately" and the like in connection with an attribute or a value particularly also define exactly the attribute or exactly the value, respectively. The term "about" in the context of a given numerate value or range refers to a value or range that is, e.g., within 20%, within 10%, within 5%, or within 2% of the given value or range. Components described as coupled or connected may be electrically or mechanically directly coupled, or they may be indirectly coupled via one or more intermediate components. Any reference signs in the claims should not be construed as limiting the scope.

### List of reference numbers:

- 1: sleep-based detection and intervention system
- 2: processing unit
- 3: template
- 4: earphone device
- 5: EEG cap
- 6a-h: electrodes
- 7: patient
- 8: acoustic intervention protocol
- 9: whole head topographies test persons
- 9': whole head topographies patient
- 33: template-based algorithm
- A1: artifact/arousal process
- R1: first regressor
- R2: second regressor

## Claims

1. A sleep-based detection and intervention system (1) for treatment of major depressive disorder comprising:
an electroencephalography cap (5) with multiple electrodes (6a, 6b, 6c, 6d, 6e, 6f, 6g, 6h) configured for recording an electroencephalogram of a patient's brain,
an earphone device (4) configured for an ear or the ears of the patient (7),
a processing unit (2) being connected to the electroencephalography cap (5) and to the earphone device (4), wherein the processing unit (2) includes
a template-based algorithm (33) configured for detecting slow wave sleep of the patient (7) based on the electroencephalogram recorded by the multiple electrodes (6a, 6b, 6c, 6d, 6e, 6f, 6g, 6h) of the electroencephalography cap (5), and
an acoustic intervention protocol (8) configured for providing a noise stimulation to the patient (7) via the earphone device (4) when slow wave sleep is detected by the template-based algorithm (33).

2. The sleep-based detection and intervention system (1) according to claim 1, wherein the template-based algorithm (33) is based on a template (3) referring to averaged slow wave sleep information of multiple test persons.

3. The sleep-based detection and intervention system (1) according to claim 2, wherein the slow wave sleep information used for creating the template (3) is in the form of multiple whole head topographies (9) from each of the multiple test persons recorded at the time-point of a frontal slow wave peak in the electroencephalogram.

4. The sleep-based detection and intervention system (1) according to claim 2 or 3, wherein the template-based algorithm (33) is configured to determine a correlation of whole head topographies (9') of the patient (7) with the template (3) in order to detect slow wave sleep.

5. The sleep-based detection and intervention system (1) according to claim 4, wherein the template-based algorithm is configured to use the determined correlation of the whole head topographies (9') of the patient (7) with the template (3) in a linear model including a first regressor (R1) and a second regressor (R2).

6. The sleep-based detection and intervention system (1) according to claim 5, wherein the first regressor (R1) includes the slow oscillation power of the template correlation, the variance of the slow oscillation power of the template correlation and a relative percentage of the slow oscillation power of the template correlation.

7. The sleep-based detection and intervention system (1) according to claim 5 or 6, wherein the second regressor (R2) includes the average global gamma power of the electroencephalogram.

8. The sleep-based detection and intervention system (1) according to any one of claims 5 to 7, wherein the correlation of the whole head topographies (9') of the patient (7) with the template (3) is calculated over a moving window, respectively, wherein the SO power of the template correlation, the variance of SO power of the template correlation and the percentage of power in SO frequency band are preferably calculated across a 10 s moving window and wherein the average global gamma power of the encephalogram is preferably calculated across a 4 s moving window.

9. The sleep-based detection and intervention system (1) according to any one of claims 4 to 8, wherein the template-based algorithm (33) is configured to determine the presence of artifacts/arousals based on an artifact/arousal detection process (A1) running parallel to slow wave sleep detection.

10. The sleep-based detection and intervention system (1) according to claim 9, wherein the artifact/arousal detection process (A1) includes the voltage range of the electroencephalogram, the amplitude and the frontal delta power as well as the average global gamma power of the electroencephalogram.

11. The sleep-based detection and intervention system (1) according to claim 10, wherein the frontal amplitude and the frontal delta power and the average global gamma power of the electroencephalogram are preferably determined across a 4 s moving window and preferably checked every 0.5 s.

12. The sleep-based detection and intervention system (1) according to any one of claims 1 to 11, wherein the acoustic intervention protocol (8) provides for a randomized noise stimulation, wherein preferably the randomized noise stimulation has a randomized duration of preferably about 50 ms to about 500 ms.

13. The sleep-based detection and intervention system (1) according to claim 12, wherein the randomized noise stimulation has a linear increase of volume, preferably from about 40 dB to about 106 dB in preferably about 60s, wherein preferably the randomized noise stimulation has randomized interstimulus intervals of about 1 s to about 4 s.

14. The sleep-based detection and intervention system (1) according to claim 13, wherein the linear increase of volume is combined with random walks between preferably about +/- 2.5 dB.

15. The sleep-based detection and intervention system (1) according to claim 1 or 12 to 14, wherein the acoustic intervention protocol (8) is configured to provide that upon artifact/arousal detection noise stimulation is reset and suppressed for a period of time, preferably for about 35 s.
